# EUROPÄISCHE PATENTSCHRIFT

(11) **EP 0 703 209 B1**
(45) Veröffentlichungstag und Bekanntmachung des Hinweises auf die Patenterteilung: **17.01.2001**
(21) Anmeldenummer: 95114167.0
(22) Anmeldetag: 09.09.1995
(51) Int. Cl.: C07C 213/00, C07C 269/06, C07C 271/16, C07C 221/00, C07C 319/20, C07C 323/43, C07F 7/10, C07B 51/00, C07C 271/18

(54) **Verfahren zur Herstellung von halogenierten Alpha-Amino-Ketonen und Alpha-Amino-Alkoholen**
Process for the preparation of halogenated alpha-amino-ketones and alpha-amino-alcohols
Procédé pour la préparation d'alpha-amino-cétones et d'alpha-amino-alcohols halogénés

(30) Priorität: 23.09.1994 CH 290594; 09.12.1994 CH 373794
(43) Veröffentlichungstag der Anmeldung: 27.03.1996
(73) Patentinhaber: F. Hoffmann-La Roche AG, 4002 Basel (CH)
(72) Erfinder: Hilpert, Hans, CH-4153 Reinach (CH)
(74) Vertreter: Witte, Hubert, Dr.

(56) Entgegenhaltungen:
- EP-A- 0 432 695
- EP-A- 0 525 880
- EP-A- 0 543 343
- EP-A- 0 710 651
- TETRAHEDRON LETTERS, Bd. 30, Nr. 46, 1989, OXFORD GB, Seiten 6393-6394, XP002012283 AKIHIKO SHIRAHATA: "Novel and facile syntheses of T-butyl substituted silanes ..."
- JOURNAL OF ORGANIC CHEMISTRY, Bd. 57, Nr. 19, 1992, EASTON US, Seiten 5247-5250, XP002012284 FABIANA D' ANIELLO ET AL. : "A stereoselective method for the preparation of HIV-1 protease inhibitors..."
- JOURNAL OF THE AMERICAN CHEMICAL SOCIETY, Bd. 116, Nr. 4, 23.Februar 1994, DC US, Seiten 1316-1323, XP002012285 ANDREI W. KONRADI ET AL.: "Pinacol cross coupling of 2-(N-(Alkoxycarbonyl)amino) Aldehydes and..."
- JOURNAL OF THE CHEMICAL SOCIETY, CHEMICAL COMMUNICATIONS, Nr. 8, August 1994, LETCHWORTH GB, Seiten 969-970, XP002012286 JOS BARLUENGA ET AL.: "The first direct preparation of chiral functionalised ketones and their synthetic uses"

## Beschreibung

Die vorliegende Erfindung betrifft ein neues Verfahren zur Herstellung von halogenierten α-Aminoketonen und α-Aminoalkoholen der Formel 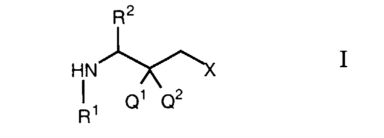 worin X Halogen, eins von Q¹ und Q² Wasserstoff und das andere Hydroxy oder Q¹ und Q² zusammen Oxo, R¹ eine Aminoschutzgruppe und R² Wasserstoff oder die charakterisierende Gruppe einer α-Aminocarbonsäure bedeuten.

Diese Verbindungen, wie sie z.B. in J. Med. Chem. 1990, 33, 1285-1288 und in der europäischen Patentpublikation 0 432 695 bzw. in dem U.S. Patent 5,196,438 beschrieben sind, sind wertvolle Zwischenprodukte zur Herstellung von pharmakologisch aktiven Verbindungen, insbesondere von solchen die sich zur Behandlung viraler Infektionen eignen.

In J. Chem. Soc., Chem. Commun. 969-970 (1994) wird von J. Barluenga et al. ein Verfahren zur Herstellung von α-Amino-halogen-ketonen aus den entsprechenden Estern sowie deren allfällige weitere Reduktion zur entsprechenden Hydroxyverbindung beschrieben. Dabei wird gemäss einem typischen Herstellungsprotokoll im letzten Absatz auf S. 969 folgendermassen vorgegangen: Ein entsprechend geschützter α-Hydroxy- oder α-Aminoester und Dihalomethan oder Chlorjodmethan in THF wurde mit Lithiumdiisopropylamid in THF oder Methyllithium in Diethylester umgesetzt und das Reaktionsgemisch aufgereinigt, sofern nicht anschliessend die Reduktion zur entsprechenden Hydroxyverbindung erfolgte.

Das erfindungsgemässe Verfahren ist dadurch gekennzeichnet, dass man
a) einen Ester der Formel 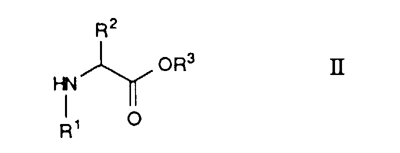 worin R³ nieder-Alkyl bedeutet und R¹ und R² obige Bedeutung haben,
   mit einem nieder-Alkyl-lithium und einem Organochlorsilan der Formel ClSi(R⁴,R⁵,R⁶), worin R⁴, R⁵ und R⁶ nieder-Alkyl oder Phenyl bedeuten, umsetzt,
b) eine intermediär gebildete silylgeschützte Verbindung der Formel 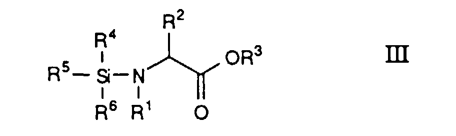 worin R¹ bis R⁶ obige Bedeutung haben,
   in Gegenwart von dihalogeniertem Methan und eines nieder-Alkyllithiums umsetzt und
c) gewünschtenfalls ein erhaltenes Keton der Formel I, worin Q¹ zusammen mit Q² Oxo bedeutet, zum Alkohol reduziert.

Im Rahmen der vorliegenden Erfindung bezeichnet Halogen, Brom, Chlor, Fluor oder Jod. Als Aminoschutzgruppen kommen solche in Frage, wie nieder-Alkoxycarbonylgruppen, Benzyloxycarbonyl, Phenoxycarbonyl oder 9-Fluorenylmethoxycarbonyl, die mit der Aminogruppe ein Carbamat bilden, oder solche wie Formyl, Acetyl oder Benzoyl, die mit der Aminogruppe ein Amid bilden, und ferner Gruppen wie Allyl oder Trityl. Der Ausdruck charakterisierende Gruppe einer α-Aminocarbonsäure bezeichnet die Gruppe R² in einer natürlichen oder synthetischen α-Aminocarbonsäure H₂NCH(R²)COOH. In natürlichen α-Aminocarbonsäuren enthaltene Gruppen R² sind Methyl (in Alanin), Isopropyl (Valin), sec.Butyl (Leucin), Methylthioäthyl (Methionin), Benzyl (Phenylalanin), 3-Indolylmethyl (Tryptophan), Hydroxymethyl (Serin), 1-Hydroxyäthyl (Threonin), Mercaptomethyl (Cystein), p-Hydroxybenzyl (Tyrosin), Carbamoylmethyl (Asparagin), Carbamoyläthyl (Glutamin), 4-Aminobutyl (Lysin), 3-Guanidinopropyl (Arginin) und 5-Imidazolylmethyl (Histidin). Als Beispiel für den Rest R² in einer synthetischen α-Aminocarbonsäure kann Cyclohexylmethyl genannt werden. Der Ausdruck "nieder-Alkyl" betrifft geradkettige oder verzweigte gesättigte Kohlenwasserstoffreste mit 1-8, vorzugsweise 1-4 Kohlenstoffatomen, wie Methyl, Aethyl, n-Propyl, Isopropyl, n-Butyl, Isobutyl, sec.Butyl, tert.Butyl, Pentyl, Hexyl, Heptyl oder Octyl.

Die Halomethylierung des Esters der Formel II zum entsprechenden Keton der Formel I, worin Q¹ und Q² zusammen Oxo bedeuten, erfolgt vorzugsweise mit in situ erzeugtem halogeniertem Methyllithium. Letzteres wird zweckmässigerweise mit dihalogeniertem Methan, vorzugsweise mit Bromchlormethan, und einem nieder-Alkyl-lithium, vorzugsweise Butyl- oder Hexyllithium, in einem Aether, vorzugsweise Tetrahydrofuran, bei -20° bis -120°C, vorzugsweise -80°C, gebildet.

Es wurde unerwarteter Weise gefunden, dass der vorgängige Schutz der im Ester der Formel II enthaltenen, bereits durch die Gruppe R¹ geschützten Aminogruppe durch eine Silylgruppe unter intermediärer Bildung der Verbindung der obigen Formel III zu einer beträchtlichen Steigerung der Ausbeute führt. Als nieder-Alkyl-lithium verwendet man vorzugsweise Butyl- oder Hexyllithium und als Organochlorsilan ClSi(R⁴,R⁵,R⁶) das Chlortrimethylsilan. Ausserdem konnte ein nahezu vollständiger Umsatz mit bedeutend weniger nieder-Alkyl-lithium und dihalogeniertem Methan erzielt werden.

Die fakultative Reduktion des erhaltenen Halomethylketons I wird zweckmässigerweise in einem Lösungsmittel wie Toluol, Tetrahydrofuran oder einem Alkohol, vorzugsweise Methanol, Aethanol oder Isopropanol, bei einer Temperatur zwischen -30 und 80°C, vorzugsweise -15°C und 50°C, ggf. unter vermindertem Druck, mittels Natrium-bis(2-metoxyäthoxy)-aluminiumhydrid, Lithium-aluminiumhydrid, Lithium-aluminium-tri-tert.-butoxyhydrid, Natrium-borhydrid, Tetramethylammonium-borhydrid oder, vorzugsweise, mittels einem Aluminium-tri-alkoxid bzw. Lithiumaluminium-tri-alkoxihydrid durchgeführt. Der Ausdruck Alkoxide umfasst gerad- oder verzweigtkettige gesättigte Kohlenwasserstoffoxide mit 1-8, vorzugsweise 3-4, Kohlenstoffatomen, nämlich Methyl-, Aethyl-, n-Propyl-, Isopropyl-, n-Butyl-, Isobutyl-, sec.Butyl-, tert. Butyloxid sowie Pentyl-, Hexyl-, Heptyl- und Octyl-Oxide. Die Aluminiumverbindungen können identische oder verschiedene Alkoxidgruppen aufweisen. Besonders bevorzugte Verbindungen sind Aluminium-tri-isopropoxid oder Aluminium-tri-sec.-butoxid. Die Reagentien Lithium-aluminium-tri-tert.butoxyhydrid, Aluminium-tri-isopropoxid und Aluminium-tri-sec.-butoxid ergaben unerwarteterweise eine hohe Stereoselektivität von 95:5 der (1S, 2S)- und (1S, 2R)-isomeren Halohydrine IV, die aus dem Reaktionsmedium in >99% optischer Reinheit und mit hoher Ausbeute kristallisiert werden konnten.

Die Ausgangsester der Formel I und die entsprechenden primären Amine, aus welchen sie abgeleitet sind, sind bekannt oder können in Analogie zu den bekannten Verbindungen hergestellt werden.

### Beispiel 1

A) Zu 800 ml Methanol wurden bei 0°C 160 ml Thionylchlorid getropft. Anschliessend wurde mit 330,4 g L-Phenylalanin versetzt und 2,5 Stunden auf 45°C erwärmt. Die entstandene Lösung wurde vollständig eingeengt, der Rückstand in 1,6 l Wasser aufgenommen und bei 0°C mit 185 ml Chlorameisensäuremethylester versetzt, wobei der pH-Wert mit 40% Natronlauge zwischen 6 - 7 gehalten wurde. Die Lösung wurde mit Toluol extrahiert, die Extrakte nach Waschen mit Wasser eingeengt und der Rückstand bei 45°C/0,1 mbar getrocknet, wobei man 474,6 g (100%) reinen (S)-2-Methoxycarbonylamino-3-phenyl-propionsäuremethylester erhielt. IR (KBr): 3338m (NH), 1726s br. (C=O), 1531s (Amid II).
B) Zu einer Lösung von 9,50 g (S)-2-Methoxycarbonylamino-3-phenylpropionsäure-methylester und 3,22 ml Bromchlormethan in 60 ml Tetrahydrofuran wurden bei -80°C 41,7 ml einer 2,6 molaren Lösung von Hexyllithium in Hexan getropft. Anschliessend wurden weitere 2,14 ml Bromchlormethan zugegeben und erneut mit 23 ml Hexyllithiumlösung versetzt, weitere 1,54 ml Bromchlormethan zugegeben und erneut mit 7,7 ml Hexyllithiumlösung versetzt. Die Lösung wurde bei -80°C mit 15 ml 20%iger methanolischer Salzsäure versetzt, auf 22° erwärmt und mit 100 ml Wasser und 40 ml Tetrahydrofuran verdünnt. Die Phasen wurden getrennt, die organische Phase mit gesättigter Natriumchloridlösung gewaschen, getrocknet und eingeengt. Der Rückstand wurde aus 40 ml Essigester und 160 ml Hexan umkristallisiert und das Kristallisat getrocknet, wobei man 3,83 g (37%) reinen (S)-(1-Benzyl-3-chlor-2-oxo-propyl)-carbaminsäure-methylester, Smp. 121° - 122°C, erhielt. IR (KBr): 3336s (NH), 1737s und 1686s (C=O), 1535s (Amid II).
C) Zu einer Lösung von 9,50 g (S)-2-Methoxycarbonylamino-3-phenyl-propionsäure-methylester in 60 ml Tetrahydrofuran wurden bei -80°C 15,4 ml einer 2,6 molaren Lösung von Hexyllithium in Hexan getropft. Anschliessend wurde mit 5,60 ml Chlortrimethylsilan versetzt. Die entstandene Suspension wurde gerührt und mit 3,22 ml Bromchlormethan versetzt. Anschliessend wurden 18,4 ml Hexyllithiumlösung zugesetzt. Die Lösung wurde bei -80°C mit 11 ml 20%iger methanolischer Salzsäure versetzt, auf 22° erwärmt und mit 100 ml Wasser und 40 ml Tetrahydrofuran verdünnt. Die Phasen wurden getrennt, die organische Phase mit gesättigter Natriumchloridlösung gewaschen, getrocknet und eingeengt. Der Rückstand wurde aus Essigester und Hexan umkristallisiert und das Kristallisat getrocknet, wobei man 7,20 g (70 %) reinen (S)-(1-Benzyl-3-chlor-2-oxo-propyl)-carbaminsäure-methylester, Smp. 122° - 123°C, erhielt. IR (KBr): 3336s (NH), 1737s und 1686s (C=O), 1535s (Amid II).
D) Variante 1
   a) Zu einer Suspension von 25,57 g (S)-(1-Benzyl-3-chlor-2-oxo-propyl)-carbaminsäure-methylester in 280 ml Aethanol wurden bei -15°C portionsweise 33.06 g Lithium-aluminium-tri-tert.butoxyhydrid gegeben und anschliessend mit 140 ml 3N Salzsäure und 170 ml Wasser bei 0°C hydrolysiert. Die Suspension wurde auf 370 ml eingeengt, abfiltriert, der Rückstand mit Wasser/Aethanol (4:1) gewaschen und getrocknet, wobei man 23.27 g (90%) isomerenreinen (1S,2S)-(1-Benzyl-3-chlor-2-hydroxy-propyl)-carbaminsäure-methylester, Smp. 163-164.5°C, erhielt. IR (KBr): 3323s, br. (NH, OH), 1689 (C=O), 1546s (Amid II).
   b) Zu einer Suspension von 21.44 g Aluminium-isopropoxid in 260 ml Isopropanol wurden bei 22°C portionsweise 25.57 g (S)-(1-Benzyl-3-chlor-2-oxo-propyl)-carbaminsäure-methylester gegeben. Die Suspension wurde bei 50°/400 mbar 2 Stunden gerührt, mit 100 ml 3N Salzsäure bei 0° hydrolysiert, anschliessend auf ein Volumen von 125 ml eingeengt, mit 125 ml Wasser verdünnt, auf 0° gekühlt und abfiltriert. Der Rückstand wurde mit Wasser/Isopropanol (4:1) gewaschen und getrocknet, wobei man 23.01 g (89%) isomerenreinen (1S,2S)-(1-Benzyl-3-chlor-2-hydroxy-propyl)-carbaminsäure-methylester, Smp. 162-163.5°C, erhielt. IR (KBr): 3323s, br. (NH, OH), 1689 (C=O), 1546s (Amid II).
D) Variante 2
   Zu einer Suspension von 46,03 g (S)-(1-Benzyl-3-chlor-2-oxo-propyl)-carbaminsäure-methylester in 275 ml Methanol wurden bei -15°C portionsweise 3,75 g Natriumborhydrid gegeben. Es wurde 1,5 Stunden gerührt, mit 21 ml Essigsäure und 460 ml Wasser verdünnt, 1 Stunde bei -15°C gerührt und abfiltriert. Der Rückstand wurde mit Wasser gewaschen, aus 430 ml Isopropanol umkristallisiert und das Kristallisat getrocknet, wobei man 28,68 g (62%) eines 98 : 2-Gemisches der (1S,2S): (1S,2R)-Isomeren des (1-Benzyl-3-chlor-2-hydroxy-propyl)-carbaminsäure-methylesters, Smp. 161,5° - 162,5°C, erhielt. IR (KBr): 3323s, br. (NH, OH), 1689s (C=O), 1546s (Amid II).

### Beispiel 2

In zu Beispiel 1 analoger Weise erhält man aus 50.8 g L-Leucinmethylesterhydrochlorid 55.3 g (97%) (S)-2-Methoxycarbonylamino-4-methyl-valeriansäuremethylester, IR (Film): 3341m (NH), 1727s br. (C=O), 1533s (Amid II),
aus 20,3 g des letzteren 15,5 g (70%) (S)-(1-Isobutyl-3-chlor-2-oxopropyl)carbaminsäuremethylester nach Umkristallisation aus Diisopropylether/Hexan, Smp. 41-42°, IR (KBr): 3321s (NH), 1739s und 1686s (C=O), 1534s (Amid II) und
aus 4,03 g des letzteren 2,32 g (57%) (1S,2S)-(1-Isobutyl-3-chlor-2-hydroxypropyl)carbaminsäuremethylester, Smp. 77-79°, IR (KBr): 3316s, br. (NH, OH), 1690s (C=O), 1549s (Amid II).

### Beispiel 3

In zu Beispiel 1 analoger Weise erhält man aus 149 g L-Methionin 221 g (100%) (S)-2-Methoxycarbonylamino-4-methylthiobuttersäuremethyl-ester,
aus 22,1 g des letzteren 16,8 g (70%) (S)-(3-Chlor-1-methylthioäthyl-2-oxopropyl)carbaminsäuremethylester und
aus 24,0 g des letzteren 20,5 g (85%) (1S,2S)-(3-Chlor-2-hydroxy-1-methylthioäthyl-propyl)carbaminsäuremethylester.

### Beispiel 4

In zu Beispiel 1 analoger Weise erhält man aus 50.0 g L-Serinmethylesterhydrochlorid 50.9 g (89%) (S)-3-Hydroxy-2-methoxycarbonylamino-propionsäure-methylester, IR (Film): 3380m, br. (NH, OH), 1722s, br. (C=O), 1534s (Amid II), aus 19.9 g des letzteren analog Beispiel 1C durch Verwendung der doppelten Menge von Hexyllithium und Chlortrimethylsilan zum Schutz der NH und OH-Gruppe 5.3 g (33%) reinen (S)-(3-Chlor-1-hydroxymethyl-2-oxo-propyl)-carbaminsäure-methylester nach Chromatographie an Kieselgel mit Hexan/Tetrahydrofuran/Methanol (5:1:1), IR (Film): 3389s, br. (NH, OH), 1706s, br. (C=O), 1529s (Amid II) und aus 2.6 g des letzteren 1.71 g (73%) (1S,2S)-(3-Chlor-2-hydroxy-1-hydroxymethyl-propyl)-carbaminsäure-methylester nach Extraktion mit Essigester, IR (Film): 3388s, br. (NH, OH), 1705s (C=O), 1529s (Amid II).

## Patentansprüche

1. Verfahren zur Herstellung von halogenierten α-Aminoketonen und α-Aminoalkoholen der Formel 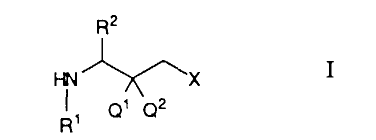 worin X Halogen, eins von Q¹ und Q² Wasserstoff und das andere Hydroxy oder Q¹ und Q² zusammen Oxo, R¹ eine Aminoschutzgruppe und R² Wasserstoff oder die charakterisierende Gruppe einer α-Aminocarbonsäure bedeuten,
dadurch gekennzeichnet, dass man
a) einen Ester der Formel 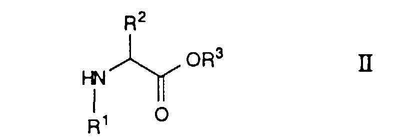 worin R³ nieder-Alkyl bedeutet und R¹ und R² obige Bedeutung haben,
mit einem nieder-Alkyl-lithium und einem Organochlorsilan der Formel ClSi(R⁴,R⁵,R⁶), worin R⁴, R⁵ und R⁶ nieder-Alkyl oder Phenyl bedeuten, umsetzt,
b) eine intermediär gebildete silylgeschützte Verbindung der Formel 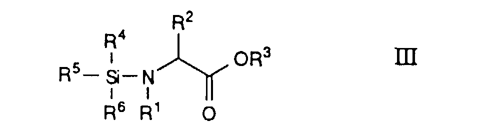 worin R¹ bis R⁶ obige Bedeutung haben,
in Gegenwart von dihalogeniertem Methan und eines nieder-Alkyllithiums umsetzt und
c) gewünschtenfalls ein erhaltenes Keton der Formel I, worin Q¹ zusammen mit Q² Oxo bedeutet, zum Alkohol reduziert.

2. Verfahren nach Anspruch 1, dadurch gekennzeichnet, dass man als Ausgangsester der Formel II und/oder als Organochlorsilan der Formel ClSi(R⁴,R⁵,R⁶) solche verwendet, worin R¹ und R³ bzw. R⁴, R⁵ und R⁶ Methyl sind.

3. Verfahren nach Anspruch 1 oder 2, worin man als dihalogeniertem Methan Bromchlormethan und/oder als nieder-Alkyl-lithium Hexyllithium verwendet.

4. Verfahren nach einem der Ansprüche 1, 2 oder 3, worin man als Reduktionsmittel ein Aluminium-trialkoxid- bzw. Lithiumaluminiumtrialkoxyhydrid verwendet.

## Claims

1. A process for the manufacture of halogenated α-aminoketones and α-aminoalcohols of the formula 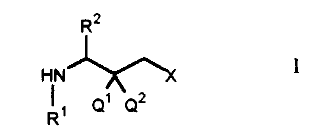 wherein X signifies halogen, one of Q¹ and Q² signifies hydrogen and the other signifies hydroxy or Q¹ and Q² together signify oxo, R' signifies an amino protecting group and R² signifies hydrogen or the characterizing group of an α-aminocarboxylic acid,
characterized by
a) reacting an ester of the formula 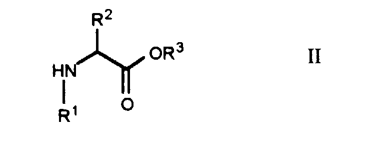 wherein R³ signifies lower-alkyl and R¹ and R² have the above significance,
with a lower-alkyl-lithium and an organochlorosilane of the formula ClSi(R⁴,R⁵,R⁶), wherein R⁴, R⁵ and R⁶ signify lower-alkyl or phenyl,
b) reacting a silyl-protected compound of the formula formed as an intermediate 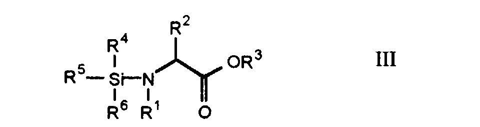 wherein R¹ to R⁶ have the above significance,
in the presence of dihalogenated methane and a lower-alkyl-lithium and
c) if desired, reducing a resulting ketone of formula I in which Q¹ and Q² together signify oxo to the alcohol.

2. A process according to claim 1, characterized in that as the starting ester of formula II and/or as the organochlorosilane of the formula ClSi(R⁴,R⁵,R⁶) there is used one in which R¹ and R³ or, respectively, R⁴, R⁵ and R⁶ are methyl.

3. A process according to claim 1 or 2, wherein bromochloromethane is used as the dihalogenated methane and/or hexyllithium is used as the lower-alkyl-lithium.

4. A process according to any one of claims claim 1, 2 or 3, wherein an aluminium trialkoxide or lithium aluminium trialkoxyhydride is used as the reducing agent.

## Revendications

1. Procédé pour la préparation d'α-amino-alcools et α-amino-cétones halogénés de formule 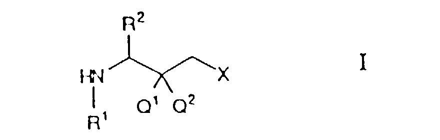 dans laquelle X représente un atome d'halogène, l'un des symboles Q¹ et Q² représente un atome d'hydrogène et l'autre représente le groupe hydroxy, ou Q¹ et Q² représentent ensemble le groupe oxo, R¹ représente un groupe protecteur de fonction amino et R² représente un atome d'hydrogène ou le groupe caractérisant d'un acide α-aminocarboxylique, caractérisé en ce que
a) on fait réagir un ester de formule 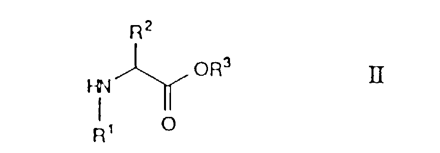 dans laquelle R³ représente un groupe alkyle inférieur et R¹ et R² ont les significations données ci-dessus,
avec un (alkyl inférieur)-lithium et un organochlorosilane de formule ClSi(R⁴,R⁵,R⁶), dans laquelle R⁴, R⁵ et R⁶ représentent un groupe alkyle inférieur ou le groupe phényle,
b) on fait réagir un composé protégé sur le groupe silyle, formé de façon intermédiaire, de formule 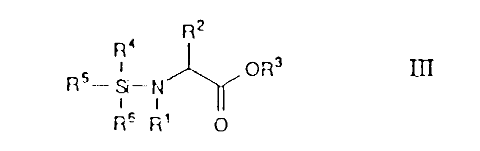 dans laquelle R¹ à R⁶ ont les significations données ci-dessus,
en présence d'un méthane dihalogéné et d'un (alkyl inférieur)-lithium et
c) si on le désire, on réduit en l'alcool une cétone obtenue de formule I, dans laquelle Q¹ représente, conjointement avec Q², le groupe oxo.

2. Procédé selon la revendication 1, caractérisé en ce que l'on utilise comme ester de formule II de départ et/ou comme organochlorosilane de formule ClSi(R⁴,R⁵,R⁶) ceux dans lesquels R¹ et R³ ou R⁴, R⁵ et R⁶ représentent le groupe méthyle.

3. Procédé selon la revendication 1 ou 2, dans lequel on utilise en tant que méthane dihalogéné le bromochlorométhane et/ou, en tant qu'(alkyl inférieur)-lithium, l'hexyl-lithium.

4. Procédé selon l'une des revendications 1, 2 et 3, dans lequel on utilise comme réducteur un hydrure de trialcoolate de lithium et d'aluminium ou de trialcoolate d'aluminium.
